# EUROPEAN PATENT APPLICATION

(11) **EP 1 002 861 A1**
(43) Date of publication of application: **24.05.2000**
(21) Application number: 99303118.6
(22) Date of filing: 22.04.1999
(51) Int. Cl.: C12N 15/11, C07K 19/00, C07K 16/46, C12N 1/19, C12N 15/81

(54) **Antigen-binding proteins comprising a linker which confers restricted conformational flexibility**

(30) Priority: 26.10.1998 WO PCT/EP98/06991
(71) Applicant: UNILEVER PLC, London EC4P 4BQ (GB)
(72) Inventor: The designation of the inventor has not yet been filed
(74) Representative: Evans, Jacqueline Gail Victoria

(57) **Abstract**

Use of a polypeptide linker group, the amino acid sequence of which group confers restricted conformational flexibility, as a linking group to link binding units, preferably antigen binding units comprising heavy chain variable domains derived from an immunoglobulin naturally devoid of light chains, in a multivalent binding protein.

SEQUENCE LISTING

<110> UNILEVER PLC
      UNILEVER N.V

<120> ANTIGEN BINDING PROTEINS

<130> T3077

<140>
<141>

<160> 40

<170> PatentIn Ver. 2.1

<210> 1
<211> 5
<212> PRT
<213> Artificial Sequence

<220>
<223> Description of Artificial Sequence:LINKER

<400> 1
Gly Thr Ser Gly Ser
  1               5

<210> 2
<211> 9
<212> PRT
<213> Artificial Sequence

<220>
<223> Description of Artificial Sequence:LINKER

<400> 2
Ser Ser Ser Ala Ser Ala Ser Ser Ala
  1               5

<210> 3
<211> 7
<212> PRT
<213> Artificial Sequence

<220>
<223> Description of Artificial Sequence:LINKER

<400> 3
Gly Ser Pro Gly Ser Pro Gly
  1               5

<210> 4

<211> 11
<212> PRT
<213> Artificial Sequence

<220>
<223> Description of Artificial Sequence:PRIMER

<400> 4
Ala Thr Thr Thr Gly Ser Ser Pro Gly Pro Thr
 1               5                   10


<210> 5
<211> 8
<212> PRT
<213> Artificial Sequence

<220>
<223> Description of Artificial Sequence:PRIMER

<400> 5
Ala Asn His Ser Gly Asn Ala Ser
 1               5


<210> 6
<211> 22
<212> DNA
<213> Artificial Sequence

<220>
<223> Description of Artificial Sequence:PRIMER

<400> 6
aggtsmarct gcagsagtcw gg                                          22


<210> 7
<211> 53
<212> DNA
<213> Artificial Sequence

<220>
<223> Description of Artificial Sequence:PRIMER

<400> 7
aacagttaag cttccgcttg cggccgcgga gctggggtct tcgctgtggt gcg        53


<210> 8
<211> 53
<212> DNA
<213> Artificial Sequence

<220>
<223> Description of Artificial Sequence:PRIMER

<400> 8

aacagttaag cttccgcttg cggccgctgg ttgtggtttt ggtgtcttgg gtt 53

<210> 9
<211> 28
<212> DNA
<213> Artificial Sequence

<220>
<223> Description of Artificial Sequence:PRIMER

<400> 9
gggaattcca ataggtggtt agcaatcg 28

<210> 10
<211> 26
<212> DNA
<213> Artificial Sequence

<220>
<223> Description of Artificial Sequence:PRIMER

<400> 10
gaccaacgtg gtcgcctggc aaaacg 26

<210> 11
<211> 26
<212> DNA
<213> Artificial Sequence

<220>
<223> Description of Artificial Sequence:PRIMER

<400> 11
cgttttgcca ggcgaccacg ttggtc 26

<210> 12
<211> 30
<212> DNA
<213> Artificial Sequence

<220>
<223> Description of Artificial Sequence:PRIMER

<400> 12
ccccaagctt acatggtctt aagttggcgt 30

<210> 13
<211> 155
<212> DNA
<213> Artificial Sequence

<220>
<223> Description of Artificial Sequence:PLASMID

CONSTRUCT

<220>
<221> CDS
<222> (3)..(140)

<400> 13
ga gct cat cac aca aac aaa caa aac aaa atg atg ctt ttg caa gcc     47
   Ala His His Thr Asn Lys Gln Asn Lys Met Met Leu Leu Gln Ala
     1               5                  10                  15

ttc ctt ttc ctt ttg gct ggt ttt gca gcc aaa ata tct gcg cag gtg     95
Phe Leu Phe Leu Leu Ala Gly Phe Ala Ala Lys Ile Ser Ala Gln Val
                 20                  25                  30

cag ctg cag gag tca taa tga ggg acc cag gtc acc gtc tcc tca        140
Gln Leu Gln Glu Ser         Gly Thr Gln Val Thr Val Ser Ser
             35                  40                  45

taatgactta agctt                                                   155


<210> 14
<211> 46
<212> PRT
<213> Artificial Sequence
<223> Description of Artificial Sequence:PLASMID
      CONSTRUCT

<400> 14
Ala His His Thr Asn Lys Gln Asn Lys Met Met Leu Leu Gln Ala Phe
  1               5                  10                  15

Leu Phe Leu Leu Ala Gly Phe Ala Ala Lys Ile Ser Ala Gln Val Gln
             20                  25                  30

Leu Gln Glu Ser         Gly Thr Gln Val Thr Val Ser Ser
         35                  40                  45


<210> 15
<211> 188
<212> DNA
<213> Artificial Sequence

<220>
<223> Description of Artificial Sequence:PLASMID
      CONSTRUCT

<220>
<221> CDS
<222> (3)..(173)

<400> 15
ga gct cat cac aca aac aaa caa aac aaa atg atg ctt ttg caa gcc     47
   Ala His His Thr Asn Lys Gln Asn Lys Met Met Leu Leu Gln Ala
     1               5                  10                  15

ttc ctt ttc ctt ttg gct ggt ttt gca gcc aaa ata tct gcg cag gtg     95
Phe Leu Phe Leu Leu Ala Gly Phe Ala Ala Lys Ile Ser Ala Gln Val
                20                  25                  30

cag ctg cag gag tca taa tga ggg acc cag gtc acc gtc tcc tca gaa     143
Gln Leu Gln Glu Ser         Gly Thr Gln Val Thr Val Ser Ser Glu
            35                  40                  45

caa aaa ctc atc tca gaa gag gat ctg aat taatgactta agctt            188
Gln Lys Leu Ile Ser Glu Glu Asp Leu Asn
        50                  55


<210> 16
<211> 57
<212> PRT
<213> Artificial Sequence
<223> Description of Artificial Sequence:PLASMID
      CONSTRUCT

<400> 16
Ala His His Thr Asn Lys Gln Asn Lys Met Met Leu Leu Gln Ala Phe
  1               5                  10                  15

Leu Phe Leu Leu Ala Gly Phe Ala Ala Lys Ile Ser Ala Gln Val Gln
            20                  25                  30

Leu Gln Glu Ser         Gly Thr Gln Val Thr Val Ser Ser Glu Gln
        35                  40                  45

Lys Leu Ile Ser Glu Glu Asp Leu Asn .
    50                  55


<210> 17
<211> 342
<212> DNA
<213> Artificial Sequence

<220>
<223> Description of Artificial Sequence:PLASMID
      CONSTRUCT

<220>
<221> CDS
<222> (1)..(342)

<400> 17
cag gtg cag ctg cag gag tca ggg gga ggc ttg gtg cag gct ggg gag     48
Gln Val Gln Leu Gln Glu Ser Gly Gly Gly Leu Val Gln Ala Gly Glu
  1               5                  10                  15

tct ctg aaa ctc tcc tgt gca gcc tct gga aac acc ttc agt ggc ggc     96
Ser Leu Lys Leu Ser Cys Ala Ala Ser Gly Asn Thr Phe Ser Gly Gly
                20                  25                  30

ttc atg ggc tgg tac cgc cag gct cca ggg aag cag cgc gag ttg gtc     144
Phe Met Gly Trp Tyr Arg Gln Ala Pro Gly Lys Gln Arg Glu Leu Val

35                  40                  45

gca acc att aat agt aga ggt atc aca aac tat gca gac ttc gtg aag    192
Ala Thr Ile Asn Ser Arg Gly Ile Thr Asn Tyr Ala Asp Phe Val Lys
    50                  55                  60

ggc cga ttc acc atc tcc aga gac aat gcc aag aag aca gtg tat ttg    240
Gly Arg Phe Thr Ile Ser Arg Asp Asn Ala Lys Lys Thr Val Tyr Leu
65                  70                  75                  80

gaa atg aac agc ctg gaa cct gaa gac acg gcc gtt tat tac tgt tac    288
Glu Met Asn Ser Leu Glu Pro Glu Asp Thr Ala Val Tyr Tyr Cys Tyr
                85                  90                  95

act cac tac ttc aga tcc tac tgg ggt cag ggg acc cag gtc acc gtc    336
Thr His Tyr Phe Arg Ser Tyr Trp Gly Gln Gly Thr Gln Val Thr Val
            100                 105                 110

tcc tca                                                            342
Ser Ser

<210> 18
<211> 114
<212> PRT
<213> Artificial Sequence
<223> Description of Artificial Sequence:PLASMID
      CONSTRUCT

<400> 18
Gln Val Gln Leu Gln Glu Ser Gly Gly Gly Leu Val Gln Ala Gly Glu
1               5                   10                  15

Ser Leu Lys Leu Ser Cys Ala Ala Ser Gly Asn Thr Phe Ser Gly Gly
            20                  25                  30

Phe Met Gly Trp Tyr Arg Gln Ala Pro Gly Lys Gln Arg Glu Leu Val
        35                  40                  45

Ala Thr Ile Asn Ser Arg Gly Ile Thr Asn Tyr Ala Asp Phe Val Lys
    50                  55                  60

Gly Arg Phe Thr Ile Ser Arg Asp Asn Ala Lys Lys Thr Val Tyr Leu
65                  70                  75                  80

Glu Met Asn Ser Leu Glu Pro Glu Asp Thr Ala Val Tyr Tyr Cys Tyr
                85                  90                  95

Thr His Tyr Phe Arg Ser Tyr Trp Gly Gln Gly Thr Gln Val Thr Val
            100                 105                 110

Ser Ser

<210> 19
<211> 351
<212> DNA
<213> Artificial Sequence

<220>
<223> Description of Artificial Sequence:PLASMID
      CONSTRUCT

<220>
<221> CDS
<222> (1)..(351)

<400> 19
cag gtg cag ctg cag gag tca ggg gga gga ttg gtg cag gcg ggg ggc      48
Gln Val Gln Leu Gln Glu Ser Gly Gly Gly Leu Val Gln Ala Gly Gly
  1               5                  10                  15

tct ctg aga ctc tcc tgt gca gcc tct gga cgc acc ggc agt acg tat      96
Ser Leu Arg Leu Ser Cys Ala Ala Ser Gly Arg Thr Gly Ser Thr Tyr
             20                  25                  30

gac atg ggc tgg ttc cgc cag gct cca ggg aag gag cgt gag tct gta      144
Asp Met Gly Trp Phe Arg Gln Ala Pro Gly Lys Glu Arg Glu Ser Val
         35                  40                  45

gca gct att aac tgg gat agt gcg cgc aca tac tat gca agc tcc gtg      192
Ala Ala Ile Asn Trp Asp Ser Ala Arg Thr Tyr Tyr Ala Ser Ser Val
     50                  55                  60

agg ggc cga ttc acc atc tcc aga gac aac gcc aag aag acg gtg tat      240
Arg Gly Arg Phe Thr Ile Ser Arg Asp Asn Ala Lys Lys Thr Val Tyr
 65                  70                  75                  80

ctg caa atg aac agc ctg aaa cct gag gac acg gcc gtt tat acc tgt      288
Leu Gln Met Asn Ser Leu Lys Pro Glu Asp Thr Ala Val Tyr Thr Cys
                 85                  90                  95

ggc gcg ggg gaa ggt ggt act tgg gac tcc tgg ggc cag ggg acc cag      336
Gly Ala Gly Glu Gly Gly Thr Trp Asp Ser Trp Gly Gln Gly Thr Gln
            100                 105                 110

gtc acc gtc tcc tca                                                  351
Val Thr Val Ser Ser
        115


<210> 20
<211> 117
<212> PRT
<213> Artificial Sequence
<223> Description of Artificial Sequence:PLASMID
      CONSTRUCT

<400> 20
Gln Val Gln Leu Gln Glu Ser Gly Gly Gly Leu Val Gln Ala Gly Gly
  1               5                  10                  15

Ser Leu Arg Leu Ser Cys Ala Ala Ser Gly Arg Thr Gly Ser Thr Tyr
             20                  25                  30

Asp Met Gly Trp Phe Arg Gln Ala Pro Gly Lys Glu Arg Glu Ser Val

35                  40                  45

Ala Ala Ile Asn Trp Asp Ser Ala Arg Thr Tyr Tyr Ala Ser Ser Val
    50                  55                  60

Arg Gly Arg Phe Thr Ile Ser Arg Asp Asn Ala Lys Lys Thr Val Tyr
65                  70                  75                  80

Leu Gln Met Asn Ser Leu Lys Pro Glu Asp Thr Ala Val Tyr Thr Cys
                85                  90                  95

Gly Ala Gly Glu Gly Gly Thr Trp Asp Ser Trp Gly Gln Gly Thr Gln
            100                 105                 110

Val Thr Val Ser Ser
        115

<210> 21
<211> 43
<212> DNA
<213> Artificial Sequence

<220>
<223> Description of Artificial Sequence:PRIMER

<400> 21
gaattaagcg gccgcccagg tgaaactgct cgagtcwggg gga                    43

<210> 22
<211> 42
<212> DNA
<213> Artificial Sequence

<220>
<223> Description of Artificial Sequence:PRIMER

<400> 22
ccctgggtcc agtggcagag gagtggcaga ggagtcttgt tt                     42

<210> 23
<211> 24
<212> DNA
<213> Artificial Sequence

<220>
<223> Description of Artificial Sequence:PRIMER

<400> 23
caggtccagc tgcaggagtc tggg                                         24

<210> 24
<211> 24
<212> DNA
<213> Artificial Sequence

<220>
<223> Description of Artificial Sequence:PRIMER

<400> 24
caggtgaaac tgctcgagtc wggg                                              24

<210> 25
<211> 55
<212> DNA
<213> Artificial Sequence

<220>
<223> Description of Artificial Sequence:LINKER;DOUBLE
      STRANDED

<220>
<221> CDS
<222> (2)..(55)

<400> 25
g gtc acc gtc tcc tca cag gtg cag ctg cag gag tca ctg taa tga ctt 49
  Val Thr Val Ser Ser Gln Val Gln Leu Gln Glu Ser Leu         Leu
    1               5                  10                  15

aag ctt                                                           55
Lys Leu

<210> 26
<211> 18
<212> PRT
<213> Artificial Sequence
<223> Description of Artificial Sequence:LINKER;DOUBLE
      STRANDED

<400> 26
Val Thr Val Ser Ser Gln Val Gln Leu Gln Glu Ser Leu         Leu
  1               5                  10                  15

Lys Leu

<210> 27
<211> 672
<212> DNA
<213> Artificial Sequence

<220>
<223> Description of Artificial Sequence:PLASMID
      CONSTRUCT

<220>
<221> CDS
<222> (1)..(672)

<400> 27
ctc gag tca ggg gga gga ttg gtg cag gcg ggg ggc tct ctg aga ctc     48
Leu Glu Ser Gly Gly Gly Leu Val Gln Ala Gly Gly Ser Leu Arg Leu
1               5                   10                  15

tcc tgt gca gcc tct gga cgc acc ggc agt acg tat gac atg ggc tgg     96
Ser Cys Ala Ala Ser Gly Arg Thr Gly Ser Thr Tyr Asp Met Gly Trp
            20                  25                  30

ttc cgc cag gct cca ggg aag gag cgt gag tct gta gca gct att aac     144
Phe Arg Gln Ala Pro Gly Lys Glu Arg Glu Ser Val Ala Ala Ile Asn
        35                  40                  45

tgg gat agt gcg cgc aca tac tat gca agc tcc gtg agg ggc cga ttc     192
Trp Asp Ser Ala Arg Thr Tyr Tyr Ala Ser Ser Val Arg Gly Arg Phe
    50                  55                  60

acc atc tcc aga gac aac gcc aag aag acg gtg tat ctg caa atg aac     240
Thr Ile Ser Arg Asp Asn Ala Lys Lys Thr Val Tyr Leu Gln Met Asn
65                  70                  75                  80

agc ctg aaa cct gag gac acg gcc gtt tat acc tgt ggc gcg ggg gaa     288
Ser Leu Lys Pro Glu Asp Thr Ala Val Tyr Thr Cys Gly Ala Gly Glu
                85                  90                  95

ggt ggt act tgg gac tcc tgg ggc cag ggg acc cag gtc acc gtc tcc     336
Gly Gly Thr Trp Asp Ser Trp Gly Gln Gly Thr Gln Val Thr Val Ser
            100                 105                 110

tca cag gtg cag ctg cag gag tca ggg gga ggc ttg gtg cag gct ggg     384
Ser Gln Val Gln Leu Gln Glu Ser Gly Gly Gly Leu Val Gln Ala Gly
        115                 120                 125

gag tct ctg aaa ctc tcc tgt gca gcc tct gga aac acc ttc agt ggc     432
Glu Ser Leu Lys Leu Ser Cys Ala Ala Ser Gly Asn Thr Phe Ser Gly
    130                 135                 140

ggc ttc atg ggc tgg tac cgc cag gct cca ggg aag cag cgc gag ttg     480
Gly Phe Met Gly Trp Tyr Arg Gln Ala Pro Gly Lys Gln Arg Glu Leu
145                 150                 155                 160

gtc gca acc att aat agt aga ggt atc aca aac tat gca gac ttc gtg     528
Val Ala Thr Ile Asn Ser Arg Gly Ile Thr Asn Tyr Ala Asp Phe Val
                165                 170                 175

aag ggc cga ttc acc atc tcc aga gac aat gcc aag aag aca gtg tat     576
Lys Gly Arg Phe Thr Ile Ser Arg Asp Asn Ala Lys Lys Thr Val Tyr
            180                 185                 190

ttg gaa atg aac agc ctg gaa cct gaa gac acg gcc gtt tat tac tgt     624
Leu Glu Met Asn Ser Leu Glu Pro Glu Asp Thr Ala Val Tyr Tyr Cys
        195                 200                 205

tac act cac tac ttc aga tcc tac tgg ggt cag ggg acc cag gtc acc     672
Tyr Thr His Tyr Phe Arg Ser Tyr Trp Gly Gln Gly Thr Gln Val Thr
    210                 215                 220

<210> 28
<211> 224
<212> PRT
<213> Artificial Sequence
<223> Description of Artificial Sequence:PLASMID
      CONSTRUCT

<400> 28
Leu Glu Ser Gly Gly Gly Leu Val Gln Ala Gly Gly Ser Leu Arg Leu
 1               5                  10                  15

Ser Cys Ala Ala Ser Gly Arg Thr Gly Ser Thr Tyr Asp Met Gly Trp
            20                  25                  30

Phe Arg Gln Ala Pro Gly Lys Glu Arg Glu Ser Val Ala Ala Ile Asn
        35                  40                  45

Trp Asp Ser Ala Arg Thr Tyr Tyr Ala Ser Ser Val Arg Gly Arg Phe
    50                  55                  60

Thr Ile Ser Arg Asp Asn Ala Lys Lys Thr Val Tyr Leu Gln Met Asn
 65                  70                  75                  80

Ser Leu Lys Pro Glu Asp Thr Ala Val Tyr Thr Cys Gly Ala Gly Glu
                85                  90                  95

Gly Gly Thr Trp Asp Ser Trp Gly Gln Gly Thr Gln Val Thr Val Ser
            100                 105                 110

Ser Gln Val Gln Leu Gln Glu Ser Gly Gly Gly Leu Val Gln Ala Gly
        115                 120                 125

Glu Ser Leu Lys Leu Ser Cys Ala Ala Ser Gly Asn Thr Phe Ser Gly
    130                 135                 140

Gly Phe Met Gly Trp Tyr Arg Gln Ala Pro Gly Lys Gln Arg Glu Leu
145                 150                 155                 160

Val Ala Thr Ile Asn Ser Arg Gly Ile Thr Asn Tyr Ala Asp Phe Val
                165                 170                 175

Lys Gly Arg Phe Thr Ile Ser Arg Asp Asn Ala Lys Lys Thr Val Tyr
            180                 185                 190

Leu Glu Met Asn Ser Leu Glu Pro Glu Asp Thr Ala Val Tyr Tyr Cys
        195                 200                 205

Tyr Thr His Tyr Phe Arg Ser Tyr Trp Gly Gln Gly Thr Gln Val Thr
    210                 215                 220


<210> 29
<211> 48
<212> DNA
<213> Artificial Sequence

<220>
<223> Description of Artificial Sequence:SYNTHETIC

INSERT

<400> 29
gtcaccgtct ctagatggcc accaggtgca gctgcaggag tcaactta 48

<210> 30
<211> 47
<212> DNA
<213> Artificial Sequence

<220>
<223> Description of Artificial Sequence:SYNTHETIC
INSERT

<400> 30
gcagagatct accggtggtc cacgtcgagc tcctcagttg aattcga 47

<210> 31
<211> 23
<212> DNA
<213> Artificial Sequence

<220>
<223> Description of Artificial Sequence:LINKER

<400> 31
ctagtggtac ttccggttcc cag 23

<210> 32
<211> 16
<212> DNA
<213> Artificial Sequence

<220>
<223> Description of Artificial Sequence:LINKER

<400> 32
accatgaagg ccaagg 16

<210> 33
<211> 35
<212> DNA
<213> Artificial Sequence

<220>
<223> Description of Artificial Sequence:LINKER

<400> 33
ctagttcttc atctgcttct gcctcttcag cccag 35

<210> 34
<211> 28
<212> DNA

<213> Artificial Sequence

<220>
<223> Description of Artificial Sequence:LINKER

<400> 34
aagaagtaga cgaagacgga gaagtcgg                                          28


<210> 35
<211> 29
<212> DNA
<213> Artificial Sequence

<220>
<223> Description of Artificial Sequence:LINKER

<400> 35
ctagtggttc tccaggttca ccaggtcag                                         29


<210> 36
<211> 22
<212> DNA
<213> Artificial Sequence

<220>
<223> Description of Artificial Sequence:LINKER

<400> 36
accaagaggt ccaagtggtc ca                                                22


<210> 37
<211> 41
<212> DNA
<213> Artificial Sequence

<220>
<223> Description of Artificial Sequence:LINKER

<400> 37
ctagtgctac tacaactggt tcttcaccag gtccaactca g                           41


<210> 38
<211> 34
<212> DNA
<213> Artificial Sequence

<220>
<223> Description of Artificial Sequence:LINKER

<400> 38
acgatgatgt tgaccaagaa gtggtccagg ttga                                   34


<210> 39

<211> 32
<212> DNA
<213> Artificial Sequence

<220>
<223> Description of Artificial Sequence:LINKER

<400> 39
ctagtgctaa tcattctggt aatgcttctc ag                                32

<210> 40
<211> 25
<212> DNA
<213> Artificial Sequence

<220>
<223> Description of Artificial Sequence:LINKER

<400> 40
acgattagta agaccattac gaaga                                        25

SEQUENCE LISTING

<110> UNILEVER PLC
      UNILEVER N.V

<120> ANTIGEN BINDING PROTEINS

<130> T3077

<140>
<141>

<160> 45

<170> PatentIn Ver. 2.1

<210> 1
<211> 5
<212> PRT
<213> Artificial Sequence

<220>
<223> Description of Artificial Sequence:LINKER

<400> 1
Gly Thr Ser Gly Ser
  1               5


<210> 2
<211> 9
<212> PRT
<213> Artificial Sequence

<220>
<223> Description of Artificial Sequence:LINKER

<400> 2
Ser Ser Ser Ala Ser Ala Ser Ser Ala
  1               5


<210> 3
<211> 7
<212> PRT
<213> Artificial Sequence

<220>
<223> Description of Artificial Sequence:LINKER

<400> 3
Gly Ser Pro Gly Ser Pro Gly
  1               5


<210> 4
<211> 11
<212> PRT
<213> Artificial Sequence

<220>
<223> Description of Artificial Sequence:PRIMER

<400> 4

Ala Thr Thr Thr Gly Ser Ser Pro Gly Pro Thr
  1               5                   10

<210> 5
<211> 8
<212> PRT
<213> Artificial Sequence

<220>
<223> Description of Artificial Sequence:PRIMER

<400> 5
Ala Asn His Ser Gly Asn Ala Ser
  1               5

<210> 6
<211> 22
<212> DNA
<213> Artificial Sequence

<220>
<223> Description of Artificial Sequence:PRIMER

<400> 6
aggtsmarct gcagsagtcw gg                                                22

<210> 7
<211> 53
<212> DNA
<213> Artificial Sequence

<220>
<223> Description of Artificial Sequence:PRIMER

<400> 7
aacagttaag cttccgcttg cggccgcgga gctggggtct tcgctgtggt gcg              53

<210> 8
<211> 53
<212> DNA
<213> Artificial Sequence

<220>
<223> Description of Artificial Sequence:PRIMER

<400> 8
aacagttaag cttccgcttg cggccgctgg ttgtggtttt ggtgtcttgg gtt              53

<210> 9
<211> 28
<212> DNA
<213> Artificial Sequence

<220>
<223> Description of Artificial Sequence:PRIMER

<400> 9
gggaattcca ataggtggtt agcaatcg                                          28

<210> 10
<211> 26
<212> DNA
<213> Artificial Sequence

<220>
<223> Description of Artificial Sequence:PRIMER

<400> 10
gaccaacgtg gtcgcctggc aaaacg                                          26


<210> 11
<211> 26
<212> DNA
<213> Artificial Sequence

<220>
<223> Description of Artificial Sequence:PRIMER

<400> 11
cgttttgcca ggcgaccacg ttggtc                                          26


<210> 12
<211> 30
<212> DNA
<213> Artificial Sequence

<220>
<223> Description of Artificial Sequence:PRIMER

<400> 12
ccccaagctt acatggtctt aagttggcgt                                      30


<210> 13
<211> 155
<212> DNA
<213> Artificial Sequence

<220>
<223> Description of Artificial Sequence:PLASMID
      CONSTRUCT

<220>
<221> CDS
<222> (3)..(110)

<220>
<221> CDS
<222> (117)..(140)

<220>
<221> CDS
<222> (147)..(155)

<400> 13
ga gct cat cac aca aac aaa caa aac aaa atg atg ctt ttg caa gcc     47
   Ala His His Thr Asn Lys Gln Asn Lys Met Met Leu Leu Gln Ala
     1               5                  10                  15

ttc ctt ttc ctt ttg gct ggt ttt gca gcc aaa ata tct gcg cag gtg    95

Phe Leu Phe Leu Leu Ala Gly Phe Ala Ala Lys Ile Ser Ala Gln Val
                20                  25                  30

cag ctg cag gag tca taatga ggg acc cag gtc acc gtc tcc tca taatga 146
Gln Leu Gln Glu Ser        Gly Thr Gln Val Thr Val Ser Ser
            35                          40

ctt aag ctt                                                       155
Leu Lys Leu
45


<210> 14
<211> 36
<212> PRT
<213> Artificial Sequence
<223> Description of Artificial Sequence:PLASMID
      CONSTRUCT

<400> 14
Ala His His Thr Asn Lys Gln Asn Lys Met Met Leu Leu Gln Ala Phe
  1               5                  10                  15

Leu Phe Leu Leu Ala Gly Phe Ala Ala Lys Ile Ser Ala Gln Val Gln
            20                  25                  30

Leu Gln Glu Ser
        35


<210> 15
<211> 8
<212> PRT
<213> Artificial Sequence
<223> Description of Artificial Sequence:PLASMID
      CONSTRUCT

<400> 15
Gly Thr Gln Val Thr Val Ser Ser
  1               5


<210> 16
<211> 3
<212> PRT
<213> Artificial Sequence
<223> Description of Artificial Sequence:PLASMID
      CONSTRUCT

<400> 16
Leu Lys Leu
  1


<210> 17
<211> 188
<212> DNA
<213> Artificial Sequence

<220>
<223> Description of Artificial Sequence:PLASMID
      CONSTRUCT

<220>

<221> CDS
<222> (3)..(110)

<220>
<221> CDS
<222> (117)..(173)

<220>
<221> CDS
<222> (180)..(188)

<400> 17
ga gct cat cac aca aac aaa caa aac aaa atg atg ctt ttg caa gcc     47
   Ala His His Thr Asn Lys Gln Asn Lys Met Met Leu Leu Gln Ala
     1               5                  10                  15

ttc ctt ttc ctt ttg gct ggt ttt gca gcc aaa ata tct gcg cag gtg     95
Phe Leu Phe Leu Leu Ala Gly Phe Ala Ala Lys Ile Ser Ala Gln Val
                20                  25                  30

cag ctg cag gag tca taatga ggg acc cag gtc acc gtc tcc tca gaa     143
Gln Leu Gln Glu Ser        Gly Thr Gln Val Thr Val Ser Ser Glu
            35                          40                  45

caa aaa ctc atc tca gaa gag gat ctg aat taatga ctt aag ctt         188
Gln Lys Leu Ile Ser Glu Glu Asp Leu Asn        Leu Lys Leu
                50                  55


<210> 18
<211> 36
<212> PRT
<213> Artificial Sequence
<223> Description of Artificial Sequence:PLASMID
      CONSTRUCT

<400> 18
Ala His His Thr Asn Lys Gln Asn Lys Met Met Leu Leu Gln Ala Phe
  1               5                  10                  15

Leu Phe Leu Leu Ala Gly Phe Ala Ala Lys Ile Ser Ala Gln Val Gln
             20                  25                  30

Leu Gln Glu Ser
         35


<210> 19
<211> 19
<212> PRT
<213> Artificial Sequence
<223> Description of Artificial Sequence:PLASMID
      CONSTRUCT

<400> 19
Gly Thr Gln Val Thr Val Ser Ser Glu Gln Lys Leu Ile Ser Glu Glu
  1               5                  10                  15

Asp Leu Asn


<210> 20
<211> 3
<212> PRT

<213> Artificial Sequence
<223> Description of Artificial Sequence:PLASMID
      CONSTRUCT

<400> 20
Leu Lys Leu
  1


<210> 21
<211> 342
<212> DNA
<213> Artificial Sequence

<220>
<223> Description of Artificial Sequence:PLASMID
      CONSTRUCT

<220>
<221> CDS
<222> (1)..(342)

<400> 21
cag gtg cag ctg cag gag tca ggg gga ggc ttg gtg cag gct ggg gag    48
Gln Val Gln Leu Gln Glu Ser Gly Gly Gly Leu Val Gln Ala Gly Glu
  1               5                  10                  15

tct ctg aaa ctc tcc tgt gca gcc tct gga aac acc ttc agt ggc ggc    96
Ser Leu Lys Leu Ser Cys Ala Ala Ser Gly Asn Thr Phe Ser Gly Gly
             20                  25                  30

ttc atg ggc tgg tac cgc cag gct cca ggg aag cag cgc gag ttg gtc    144
Phe Met Gly Trp Tyr Arg Gln Ala Pro Gly Lys Gln Arg Glu Leu Val
         35                  40                  45

gca acc att aat agt aga ggt atc aca aac tat gca gac ttc gtg aag    192
Ala Thr Ile Asn Ser Arg Gly Ile Thr Asn Tyr Ala Asp Phe Val Lys
     50                  55                  60

ggc cga ttc acc atc tcc aga gac aat gcc aag aag aca gtg tat ttg    240
Gly Arg Phe Thr Ile Ser Arg Asp Asn Ala Lys Lys Thr Val Tyr Leu
 65                  70                  75                  80

gaa atg aac agc ctg gaa cct gaa gac acg gcc gtt tat tac tgt tac    288
Glu Met Asn Ser Leu Glu Pro Glu Asp Thr Ala Val Tyr Tyr Cys Tyr
                 85                  90                  95

act cac tac ttc aga tcc tac tgg ggt cag ggg acc cag gtc acc gtc    336
Thr His Tyr Phe Arg Ser Tyr Trp Gly Gln Gly Thr Gln Val Thr Val
            100                 105                 110

tcc tca                                                            342
Ser Ser


<210> 22
<211> 114
<212> PRT
<213> Artificial Sequence
<223> Description of Artificial Sequence:PLASMID
      CONSTRUCT

<400> 22
Gln Val Gln Leu Gln Glu Ser Gly Gly Gly Leu Val Gln Ala Gly Glu

1               5                   10                  15

Ser Leu Lys Leu Ser Cys Ala Ala Ser Gly Asn Thr Phe Ser Gly Gly
            20                  25                  30

Phe Met Gly Trp Tyr Arg Gln Ala Pro Gly Lys Gln Arg Glu Leu Val
        35                  40                  45

Ala Thr Ile Asn Ser Arg Gly Ile Thr Asn Tyr Ala Asp Phe Val Lys
    50                  55                  60

Gly Arg Phe Thr Ile Ser Arg Asp Asn Ala Lys Lys Thr Val Tyr Leu
65                  70                  75                  80

Glu Met Asn Ser Leu Glu Pro Glu Asp Thr Ala Val Tyr Tyr Cys Tyr
                85                  90                  95

Thr His Tyr Phe Arg Ser Tyr Trp Gly Gln Gly Thr Gln Val Thr Val
            100                 105                 110

Ser Ser

<210> 23
<211> 351
<212> DNA
<213> Artificial Sequence

<220>
<223> Description of Artificial Sequence:PLASMID
      CONSTRUCT

<220>
<221> CDS
<222> (1)..(351)

<400> 23
cag gtg cag ctg cag gag tca ggg gga gga ttg gtg cag gcg ggg ggc    48
Gln Val Gln Leu Gln Glu Ser Gly Gly Gly Leu Val Gln Ala Gly Gly
  1               5                  10                  15

tct ctg aga ctc tcc tgt gca gcc tct gga cgc acc ggc agt acg tat    96
Ser Leu Arg Leu Ser Cys Ala Ala Ser Gly Arg Thr Gly Ser Thr Tyr
             20                  25                  30

gac atg ggc tgg ttc cgc cag gct cca ggg aag gag cgt gag tct gta    144
Asp Met Gly Trp Phe Arg Gln Ala Pro Gly Lys Glu Arg Glu Ser Val
         35                  40                  45

gca gct att aac tgg gat agt gcg cgc aca tac tat gca agc tcc gtg    192
Ala Ala Ile Asn Trp Asp Ser Ala Arg Thr Tyr Tyr Ala Ser Ser Val
     50                  55                  60

agg ggc cga ttc acc atc tcc aga gac aac gcc aag aag acg gtg tat    240
Arg Gly Arg Phe Thr Ile Ser Arg Asp Asn Ala Lys Lys Thr Val Tyr
 65                  70                  75                  80

ctg caa atg aac agc ctg aaa cct gag gac acg gcc gtt tat acc tgt    288
Leu Gln Met Asn Ser Leu Lys Pro Glu Asp Thr Ala Val Tyr Thr Cys
                 85                  90                  95

ggc gcg ggg gaa ggt ggt act tgg gac tcc tgg ggc cag ggg acc cag    336
Gly Ala Gly Glu Gly Gly Thr Trp Asp Ser Trp Gly Gln Gly Thr Gln
            100                 105                 110

gtc acc gtc tcc tca                                                351
Val Thr Val Ser Ser
        115

<210> 24
<211> 117
<212> PRT
<213> Artificial Sequence
<223> Description of Artificial Sequence:PLASMID
      CONSTRUCT

<400> 24
Gln Val Gln Leu Gln Glu Ser Gly Gly Gly Leu Val Gln Ala Gly Gly
  1               5                  10                  15

Ser Leu Arg Leu Ser Cys Ala Ala Ser Gly Arg Thr Gly Ser Thr Tyr
             20                  25                  30

Asp Met Gly Trp Phe Arg Gln Ala Pro Gly Lys Glu Arg Glu Ser Val
         35                  40                  45

Ala Ala Ile Asn Trp Asp Ser Ala Arg Thr Tyr Tyr Ala Ser Ser Val
     50                  55                  60

Arg Gly Arg Phe Thr Ile Ser Arg Asp Asn Ala Lys Lys Thr Val Tyr
 65                  70                  75                  80

Leu Gln Met Asn Ser Leu Lys Pro Glu Asp Thr Ala Val Tyr Thr Cys
                 85                  90                  95

Gly Ala Gly Glu Gly Gly Thr Trp Asp Ser Trp Gly Gln Gly Thr Gln
            100                 105                 110

Val Thr Val Ser Ser
        115

<210> 25
<211> 43
<212> DNA
<213> Artificial Sequence

<220>
<223> Description of Artificial Sequence:PRIMER

<400> 25
gaattaagcg gccgcccagg tgaaactgct cgagtcwggg gga                    43

<210> 26
<211> 42
<212> DNA
<213> Artificial Sequence

<220>
<223> Description of Artificial Sequence:PRIMER

<400> 26
ccctgggtcc agtggcagag gagtggcaga ggagtcttgt tt                     42

<210> 27

<211> 24
<212> DNA
<213> Artificial Sequence

<220>
<223> Description of Artificial Sequence:PRIMER

<400> 27
caggtccagc tgcaggagtc tggg                                              24


<210> 28
<211> 24
<212> DNA
<213> Artificial Sequence

<220>
<223> Description of Artificial Sequence:PRIMER

<400> 28
caggtgaaac tgctcgagtc wggg                                              24



<210> 29
<211> 55
<212> DNA
<213> Artificial Sequence

<220>
<223> Description of Artificial Sequence:LINKER;DOUBLE
      STRANDED

<220>
<221> CDS
<222> (2)..(40)

<220>
<221> CDS
<222> (47)..(55)

<400> 29
g gtc acc gtc tcc tca cag gtg cag ctg cag gag tca ctg taatga ctt  49
  Val Thr Val Ser Ser Gln Val Gln Leu Gln Glu Ser Leu        Leu
    1               5                   10

aag ctt                                                           55
Lys Leu
15


<210> 30
<211> 13
<212> PRT
<213> Artificial Sequence
<223> Description of Artificial Sequence:LINKER;DOUBLE
      STRANDED

<400> 30
Val Thr Val Ser Ser Gln Val Gln Leu Gln Glu Ser Leu
  1               5                   10


<210> 31

<211> 3
<212> PRT
<213> Artificial Sequence
<223> Description of Artificial Sequence:LINKER;DOUBLE
      STRANDED

<400> 31
Leu Lys Leu
  1


<210> 32
<211> 672
<212> DNA
<213> Artificial Sequence

<220>
<223> Description of Artificial Sequence:PLASMID
      CONSTRUCT

<220>
<221> CDS
<222> (1)..(672)

<400> 32
ctc gag tca ggg gga gga ttg gtg cag gcg ggg ggc tct ctg aga ctc    48
Leu Glu Ser Gly Gly Gly Leu Val Gln Ala Gly Gly Ser Leu Arg Leu
  1               5                  10                  15

tcc tgt gca gcc tct gga cgc acc ggc agt acg tat gac atg ggc tgg    96
Ser Cys Ala Ala Ser Gly Arg Thr Gly Ser Thr Tyr Asp Met Gly Trp
             20                  25                  30

ttc cgc cag gct cca ggg aag gag cgt gag tct gta gca gct att aac    144
Phe Arg Gln Ala Pro Gly Lys Glu Arg Glu Ser Val Ala Ala Ile Asn
         35                  40                  45

tgg gat agt gcg cgc aca tac tat gca agc tcc gtg agg ggc cga ttc    192
Trp Asp Ser Ala Arg Thr Tyr Tyr Ala Ser Ser Val Arg Gly Arg Phe
     50                  55                  60

acc atc tcc aga gac aac gcc aag aag acg gtg tat ctg caa atg aac    240
Thr Ile Ser Arg Asp Asn Ala Lys Lys Thr Val Tyr Leu Gln Met Asn
 65                  70                  75                  80

agc ctg aaa cct gag gac acg gcc gtt tat acc tgt ggc gcg ggg gaa    288
Ser Leu Lys Pro Glu Asp Thr Ala Val Tyr Thr Cys Gly Ala Gly Glu
                 85                  90                  95

ggt ggt act tgg gac tcc tgg ggc cag ggg acc cag gtc acc gtc tcc    336
Gly Gly Thr Trp Asp Ser Trp Gly Gln Gly Thr Gln Val Thr Val Ser
            100                 105                 110

tca cag gtg cag ctg cag gag tca ggg gga ggc ttg gtg cag gct ggg    384
Ser Gln Val Gln Leu Gln Glu Ser Gly Gly Gly Leu Val Gln Ala Gly
        115                 120                 125

gag tct ctg aaa ctc tcc tgt gca gcc tct gga aac acc ttc agt ggc    432
Glu Ser Leu Lys Leu Ser Cys Ala Ala Ser Gly Asn Thr Phe Ser Gly
    130                 135                 140

ggc ttc atg ggc tgg tac cgc cag gct cca ggg aag cag cgc gag ttg    480
Gly Phe Met Gly Trp Tyr Arg Gln Ala Pro Gly Lys Gln Arg Glu Leu
145                 150                 155                 160

gtc gca acc att aat agt aga ggt atc aca aac tat gca gac ttc gtg    528
Val Ala Thr Ile Asn Ser Arg Gly Ile Thr Asn Tyr Ala Asp Phe Val
                165                 170                 175

aag ggc cga ttc acc atc tcc aga gac aat gcc aag aag aca gtg tat    576
Lys Gly Arg Phe Thr Ile Ser Arg Asp Asn Ala Lys Lys Thr Val Tyr
            180                 185                 190

ttg gaa atg aac agc ctg gaa cct gaa gac acg gcc gtt tat tac tgt    624
Leu Glu Met Asn Ser Leu Glu Pro Glu Asp Thr Ala Val Tyr Tyr Cys
        195                 200                 205

tac act cac tac ttc aga tcc tac tgg ggt cag ggg acc cag gtc acc    672
Tyr Thr His Tyr Phe Arg Ser Tyr Trp Gly Gln Gly Thr Gln Val Thr
    210                 215                 220


<210> 33
<211> 224
<212> PRT
<213> Artificial Sequence
<223> Description of Artificial Sequence:PLASMID
      CONSTRUCT

<400> 33
Leu Glu Ser Gly Gly Gly Leu Val Gln Ala Gly Gly Ser Leu Arg Leu
  1               5                  10                  15

Ser Cys Ala Ala Ser Gly Arg Thr Gly Ser Thr Tyr Asp Met Gly Trp
             20                  25                  30

Phe Arg Gln Ala Pro Gly Lys Glu Arg Glu Ser Val Ala Ala Ile Asn
         35                  40                  45

Trp Asp Ser Ala Arg Thr Tyr Tyr Ala Ser Ser Val Arg Gly Arg Phe
     50                  55                  60

Thr Ile Ser Arg Asp Asn Ala Lys Lys Thr Val Tyr Leu Gln Met Asn
 65                  70                  75                  80

Ser Leu Lys Pro Glu Asp Thr Ala Val Tyr Thr Cys Gly Ala Gly Glu
                 85                  90                  95

Gly Gly Thr Trp Asp Ser Trp Gly Gln Gly Thr Gln Val Thr Val Ser
            100                 105                 110

Ser Gln Val Gln Leu Gln Glu Ser Gly Gly Gly Leu Val Gln Ala Gly
        115                 120                 125

Glu Ser Leu Lys Leu Ser Cys Ala Ala Ser Gly Asn Thr Phe Ser Gly
    130                 135                 140

Gly Phe Met Gly Trp Tyr Arg Gln Ala Pro Gly Lys Gln Arg Glu Leu
145                 150                 155                 160

Val Ala Thr Ile Asn Ser Arg Gly Ile Thr Asn Tyr Ala Asp Phe Val
                165                 170                 175

Lys Gly Arg Phe Thr Ile Ser Arg Asp Asn Ala Lys Lys Thr Val Tyr
            180                 185                 190

Leu Glu Met Asn Ser Leu Glu Pro Glu Asp Thr Ala Val Tyr Tyr Cys
        195                 200                 205

Tyr Thr His Tyr Phe Arg Ser Tyr Trp Gly Gln Gly Thr Gln Val Thr
    210                 215                 220

<210> 34
<211> 48
<212> DNA
<213> Artificial Sequence

<220>
<223> Description of Artificial Sequence:SYNTHETIC
      INSERT

<400> 34
gtcaccgtct ctagatggcc accaggtgca gctgcaggag tcaactta                48

<210> 35
<211> 47
<212> DNA
<213> Artificial Sequence

<220>
<223> Description of Artificial Sequence:SYNTHETIC
      INSERT

<400> 35
gcagagatct accggtggtc cacgtcgagc tcctcagttg aattcga                 47

<210> 36
<211> 23
<212> DNA
<213> Artificial Sequence

<220>
<223> Description of Artificial Sequence:LINKER

<400> 36
ctagtggtac ttccggttcc cag                                           23

<210> 37
<211> 16
<212> DNA
<213> Artificial Sequence

<220>
<223> Description of Artificial Sequence:LINKER

<400> 37
accatgaagg ccaagg                                                   16

<210> 38
<211> 35
<212> DNA
<213> Artificial Sequence

<220>
<223> Description of Artificial Sequence:LINKER

<400> 38

ctagttcttc atctgcttct gcctcttcag cccag                                35

<210> 39
<211> 28
<212> DNA
<213> Artificial Sequence

<220>
<223> Description of Artificial Sequence:LINKER

<400> 39
aagaagtaga cgaagacgga gaagtcgg                                        28

<210> 40
<211> 29
<212> DNA
<213> Artificial Sequence

<220>
<223> Description of Artificial Sequence:LINKER

<400> 40
ctagtggttc tccaggttca ccaggtcag                                       29

<210> 41
<211> 22
<212> DNA
<213> Artificial Sequence

<220>
<223> Description of Artificial Sequence:LINKER

<400> 41
accaagaggt ccaagtggtc ca                                              22

<210> 42
<211> 41
<212> DNA
<213> Artificial Sequence

<220>
<223> Description of Artificial Sequence:LINKER

<400> 42
ctagtgctac tacaactggt tcttcaccag gtccaactca g                         41

<210> 43
<211> 34
<212> DNA
<213> Artificial Sequence

<220>
<223> Description of Artificial Sequence:LINKER

<400> 43
acgatgatgt tgaccaagaa gtggtccagg ttga                                 34

<210> 44

<211> 32
<212> DNA
<213> Artificial Sequence

<220>
<223> Description of Artificial Sequence:LINKER

<400> 44
ctagtgctaa tcattctggt aatgcttctc ag                                32


<210> 45
<211> 25
<212> DNA
<213> Artificial Sequence

<220>
<223> Description of Artificial Sequence:LINKER

<400> 45
acgattagta agaccattac gaaga                                        25

## Description

### FIELD OF THE INVENTION

The present invention relates to the preparation of multivalent and multispecific binding proteins. In particular, the invention relates to the preparation of antigen binding proteins comprising a plurality of binding units linked in series by means of intervening polypeptide linker groups, the amino acid sequence of which linker groups confer restricted conformational flexibility.

### BACKGROUND OF THE INVENTION

There is considerable interest in the preparation of multivalent and/or multispecific antigen binding proteins. Antigen binding proteins which are multivalent (that is, comprise more than one antigen binding site), more especially those which are also multispecific (where the antigen binding sites have differing antigen specificities) have found particular application in the fields of diagnosis or therapy, for example, where the construction of binding proteins having binding activity .against both target site and diagnostic or therapeutic agent allows for targeted delivery of the diagnostic or therapeutic agent to the intended site of action. Other uses for which multivalent and multispecific binding proteins have been proposed include assays, such as immunoassays and agglutination assays, and purification processes.

Those multivalent, multispecific antigen binding proteins which have been described in the literature to date rely, in general, on the association of antibody light and heavy chain variable domains for the formation of the antigen binding site.

Thus, constructs comprising two or more polypeptide chains are described in WO 94/09131 (Scotgen Limited) and WO 97/14719 (Unilever) and WO 97/38102 (Unilever); multivalent molecules comprising two or more single chain Fv molecules linked together are described in WO 93/11161 (Enzon Inc.) and WO 94/13806 (Dow Chemical Co.).

WO 94/04678 (Casterman et al) describes immunoglobulins capable of exhibiting the functional properties of classical, four chain, immunoglobulins but which comprise two heavy polypeptide chains only and are naturally devoid of light polypeptide chains. Fragments corresponding to isolated V 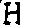 domains or to V 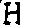 dimers linked by the hinge disulphide are also disclosed. These immunoglobulins, which may be isolated from Camelids, do not rely on the association of heavy and light chain variable domains for the formation of the antigen-binding site; instead, the heavy chain variable domain (hereinafter V 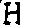 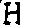 ) alone forms the complete antigen binding site, constituting a single domain binding site.

In their later patent application, WO 96/34103, Casterman et al disclose multivalent, multispecific constructs comprising V 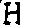 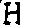 fragments combined with a linker sequence. Suitable linker sequences disclosed and exemplified are derived from sequences corresponding to the hinge domain of an immunoglobulin devoid of light chains.

In the Applicant's co-pending patent application number PCT/EP98/06991, filed 26th October 1998, there are disclosed multivalent, multispecific antigen-binding proteins comprising a polypeptide comprising in series two or more single domain binding units which are preferably variable domains of a heavy chain derived from an immunoglobulin naturally devoid of light chains. The individual single domain binding units may suitably be linked by means of peptide linkers, preferably flexible peptide linkers, which allow the variable domains to flex in relation to each other with the aim of ensuring that they can bind to multiple antigenic determinants simultaneously.

There remains a continuing need for the development of improved methods for producing multivalent and/or multispecific binding proteins, especially antigen binding proteins. In particular, there is commercial interest in producing molecules which not only have improved binding activity but which also can be produced economically on a large scale.

### SUMMARY OF THE INVENTION

In a first aspect, the invention provides the use of a polypeptide group, the amino acid sequence of which group confers restricted conformational flexibility, as a linking group to link binding units in a multivalent binding protein.

The invention also provides a multivalent binding protein comprising a plurality of binding units linked by means of intervening polypeptide linker groups, the amino acid sequence of which linker group confers restricted conformational flexibility.

The invention further provides a nucleotide sequence encoding a multivalent antigen binding protein according to the invention and cloning and expression vectors comprising such nucleotide sequences. Also provided are host cells transformed with vectors comprising such nucleotide sequences.

As used herein, a 'multivalent binding protein' is a protein which has more than one binding units which allow for specific binding with a molecule partner in a binding pair. Included within this are bivalent, trivalent and so on. Examples of suitable binding units include antigen binding domains of antibodies, binding domains of receptors such as hormone receptors, lectins, enzymes, and cell adhesion molecules. A 'multivalent antigen binding protein' is a protein which has more than one antigen binding unit.

An 'antigen binding unit' is any structure which exhibits antigen-binding activity. This may be an antibody or an immunologically active fragment thereof. An 'antibody' refers to an immunoglobulin which may be derived from natural sources or synthetically produced. Unless indicated otherwise, 'antibody' and 'immunoglobulin' are used synonymously throughout this specification.

An antibody fragment is a portion of a whole antibody which retains the ability to exhibit antigen-binding activity. The antigen binding site may be formed through association of antibody light and heavy chain variable domains or may comprise individual antibody variable domains, constituting a single domain binding site.

Suitable fragments include Fab (comprising an antibody light chain associated with the V_{H} and C_{H1} domains of an antibody heavy chain), Fv (comprising the variable domains of antibody heavy and light chains associated with each other) and scFv (comprising an antibody V_{H} domain linked to a V 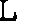 domain by a flexible peptide linker) fragments. Where the antigen binding site comprises a single variable domain, this may be a heavy chain variable domain, most suitably a heavy chain variable domain derived from an immunoglobulin naturally devoid of light chains.

'Restricted conformational flexibility' relates to restriction of movement of the antigen binding units about the backbone of the intervening polypeptide linker group.

The present invention may be more fully understood with reference to the following description, when read together with the accompanying drawings. For convenience, an antigen binding protein comprising two single binding units is hereinafter referred to as a 'bi-head'.

### BRIEF DESCRIPTION OF THE DRAWINGS

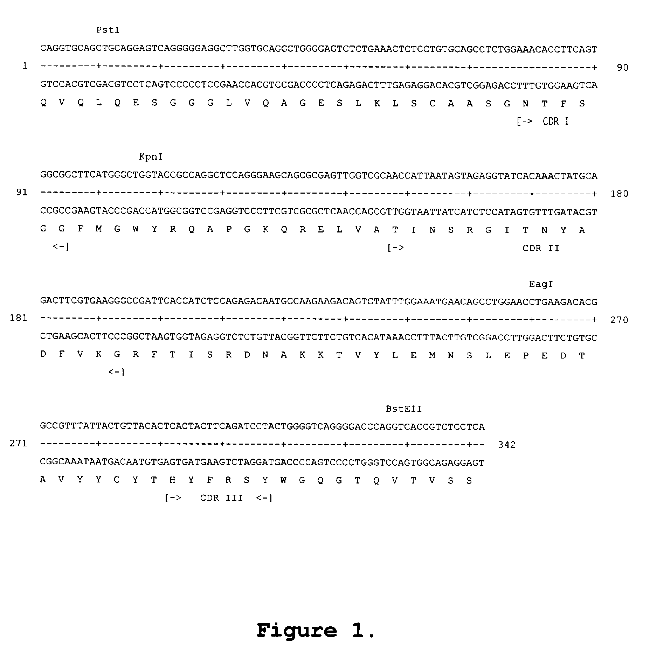

Figure 1 shows a nucleotide sequence of the PstI-BstEII insert of plasmid pUR4640, encoding the heavy chain variable domain of an anti-RR6 antibody (denoted R9) from a llama.
Figure 2 shows the nucleotide sequence of the PstI-BstEII insert of plasmid pUR4601, encoding the heavy chain variable domain of an anti-hCG antibody (denoted H14) from a llama.
Figure 3 shows a map of plasmid pUR4619.
Figure 4 shows the nucleotide sequence within plasmid pUR4619 which encodes an anti-hCG-anti-RR6 bispecific biheaded antigen-binding protein (denoted HI4-R9), missing the first 4 and last 3 amino acids.
Figure 5 shows the A405 signals of an ELISA to determine bispecificity of various HI4-R9 biheads.
Figure 6 shows the scores achieved in a rapid assay technology (RAT) format assay following the detection of 1 IU/ml hCG (human chorionic gonadotrophin protein) with various anti-hCG-anti-RR6 bihead antigen binding proteins derived from a llama wherein the anti-hCG and anti-RR6 fragments are linked as follows (see Example 1.5):

| Number | Linker | |
|---|---|---|
| 1 | no linker (directly attached) | |
| 2 | G-T-S-G-S | (SEQ. ID NO. 1) |
| 3 | S-S-S-A-S-A-S-S-A | (SEQ. ID NO. 2) |
| 4 | G-S-P-G-S-P-G | (SEQ. ID NO. 3) |
| 5 | A-T-T-T-G-S-S-P-G-P-T | (SEQ. ID NO. 4) |
| 6 | A-N-H-S-G-N-A-S | (SEQ. ID NO. 5) |

Figure 7 shows a comparison of the sensitivity of detection of hCG in a RAT assay using various biheads (see Example 1.5).

### DETAILED DESCRIPTION OF THE INVENTION

The invention is based on the unexpected finding that by using a polypeptide linking group conferring restricted conformational flexibility to link together antigen binding units, multivalent antigen binding proteins having advantageous binding affinity, as demonstrated by their increased sensitivity of diagnosis and detection, are obtained. Furthermore, constructs according to the invention may conveniently be produced at high yields economically and efficiently on a scale appropriate for industrial use.

As is apparent from the discussion of the background to the invention above, to the extent that multivalent antigen binding constructs comprising separate binding units linked together have been described at all in the literature, the linking means has been provided by flexible peptide groups. Flexibility of conformation in the linker group has been considered desirable in order to allow the multivalent construct to assume the correct orientation to allow simultaneous binding of multiple antigens.

Surprisingly, the present inventors have found that by restricting the conformational flexibility of the linking polypeptide group, multivalent antigen binding constructs having improved binding affinity may be obtained. This is entirely contrary to the teaching in the art that the linking group should desirably be flexible.

The invention is applicable to the preparation of multivalent antigen binding constructs comprising antigen binding units where the antigen binding site is formed through association of antibody light and heavy chain variable domains. Preferably, however, the constructs prepared according to the invention comprise a plurality of single domain binding units, more particularly a plurality of heavy chain variable domains derived from an immunoglobulin naturally devoid of light chains such as may be obtained from lymphoid cells, especially peripheral blood lymphocytes, bone marrow cells or spleen cells derived from Camelids as described in WO 94/04678 (Casterman et al) discussed above. An advantage of using single domain binding units which are heavy chain variable domains derived from Camelids is that they can readily and conveniently be produced economically on a large scale, for example, using a transformed lower eukaryotic host, as described in WO 94/25591 (Unilever), described above.

Bivalent forms, that is having two antigen binding sites, of the multivalent antigen binding proteins prepared according to the invention are preferred but it will be appreciated that higher multivalent forms, which are also encompassed in the present invention, may find application under suitable circumstances, for example where more than two antigens are required to bind, for example in processes for scavenging molecules from solution or processes where close proximity of molecules form the basis of an assay.

Structural features which may suitably be incorporated into the linking polypeptide group in order to achieve the effect of restricting conformational flexibility according to the purposes of the invention would readily suggest themselves to those skilled in the art.

Accordingly, in one embodiment, the linker group preferably comprises one or more proline residues.

Without wishing to be bound by theory, it is generally thought that the presence of a proline residue in a peptide sequence encourages the amino acid backbone of the peptide to adopt a beta-turn structural configuration, with the peptide backbone changing direction about the proline residue. Linker groups comprising other sequence features which promote the formation of a beta-turn configuration in the peptide backbone, such as peptide linkers containing valine residues or constrained residues such as 8-bicyclic and 5,9-bicyclic tripeptide units (see, for example, Johannesson et al, J. Med. Chem., 42, 601-608 (1999), may also suitably find application in the present invention.

In another embodiment, peptide linker groups derived from naturally occurring proteins such as cell wall proteins (CWP), in particular, CWP1, or cellobishydrolases (CBH), such as CBH1P, which serve to restrict conformational flexibility or linker groups showing at least 50% homology thereto as determined by the ALIGN program of Dayhoff et al (1983), Methods Enzymol., 91, 524-545, may also suitably be used according to the invention.

Peptide linker groups which encode a glycosylation binding site and/or are resistant to proteolytic attack may also suitably be employed. Here, the presence of a carbohydrate attached to the amino acid residues has the effect of restricting the flexibility of the peptide backbone.

Conveniently, the polypeptide linking group according to the invention comprises from 4 to 30 amino acid residues, preferably from 5 to 15 amino acid residues.

Preferred polypeptide linking groups according to the invention comprise an amino acid sequence selected from:
- S-S-S-A-S-A-S-S-A,: (SEQ. ID NO. 2)
- G-S-P-G-S-P-G,: (SEQ. ID NO. 3)
- A-T-T-T-G-S-S-P-G-P-T: (SEQ. ID NO. 4)

It will be appreciated that although the invention has been described primarily by reference to antigen binding proteins, it is equally applicable to proteins comprising other binding units as described above. References to antigen binding proteins will accordingly be understood to refer also to such other proteins unless the context dictates otherwise.

Multivalent antigen binding proteins according to the invention may be prepared by transforming a host by incorporating a gene encoding the polypeptide as set forth above and expressing said gene in said host.

Suitably the host or hosts may be selected from prokaryotic bacteria, such as Gram-negative bacteria, for example *E. coli*, and Gram-positive bacteria, for example *B. subtilis* or lactic acid bacteria, lower eukaryotes such as yeasts, for example belonging to the genera *Saccharomyces, Kluyveromyces, Hansenula* or *Pichia*, or moulds such as those belonging to the genera *Aspergillus* or *Trichoderma*.

Preferred hosts for use in connection with the present invention are the lower eukaryotic moulds and yeasts.

Techniques for synthesising genes, incorporating them into hosts and expressing genes in hosts are well known in the art and the skilled person would readily be able to put the invention into effect using common general knowledge.

Methods for producing antibody fragments or functionalised fragments thereof derived from the heavy chain immunoglobulin of *Camelidae* using a transformed lower eukaryotic host are described, for example in patent application WO 94/25591 and such techniques may suitably be applied to prepare constructs according to the present invention.

Proteins according to the invention may be recovered and purified using conventional techniques such as affinity chromatography, ion exchange chromatography or gel filtration chromatography.

The activity of the multivalent binding proteins according to the invention may conveniently be measured by standard techniques known in the art such as enzyme-linked immunoadsorbant assay (ELISA), radioimmune assay (RIA) or by using biosensors.

The following examples are produced by way of illustration only.

Techniques used for the manipulation and analysis of nucleic acid materials were performed as described in Sambrook et al, Molecular Cloning, Cold Spring Harbor Press, New York, 2nd Ed., (1989) unless otherwise indicated.

Restriction sites are underlined.
HC-V denotes heavy chain variable domain.

### EXAMPLES

### Example 1 Self Assembling Llama Bi-heads Containing Linker Peptides on Latex to Assay hCG

### 1.1 Construction of Llama Bi-heads with Various Linkers

### a) Induction of humeral immune responses in llama

Male llamas were immunised with a water in oil emulsion (1:9 V/V, antigen in water: Specol (Bokhout et al, Vet. Immunol. Immunopath., **2**:, 491-500 (1981)) subcutaneously and intramuscularly. Per immunisation site 0.75-1.5 ml water in oil emulsion was inoculated containing 100:g antigen. The antigens used were: hCG (Sigma), azo-dye RR6 (ICI) which was coupled to BSA via its reactive triazine group. Immunisations were performed according to the following time table: The second immunisation was performed three weeks after the first. The third was performed two weeks after the second immunisation. The immune response was followed by antigen specific ELISAs.

The anti-RR-6 response was measured by using Nunc Covalink plates, which where coated with the azo-dye. After incubation with (diluted) serum samples, the bound llama antibodies were detected via a incubation with poly-clonal rabbit-anti-llama antiserum (obtained via immunising rabbits with llama immunoglobulins which were purified via ProtA and ProtG columns; ID-DLO), followed by an incubation with swine-anti-rabbit immunoglobulins (Dako) conjugated with alkaline phosphatase. Finally the alkaline phosphatase enzyme-activity was determined after incubation with p-nitro-phenyl phosphate and the optical density was measured at 405nm. The anti-hCG response, was measured in essentially the same way using Nunc maxi-sorb plates coated with hCG.

### b) Cloning, expressing and screening of llama HC-V fragments

### i) Isolation of gene fragments encoding llama HC-V domains

From an immunised llama a blood sample of about 200ml was taken and an enriched lymphocyte population was obtained via Ficoll (Pharmacia) discontinuous gradient centrifugation. From these cells, total RNA was isolated by acid guanidium thiocyanate extraction (e.g. via the method described by Chomczynnski and Sacchi, Analytical Biochemistry, 162: 156-159 (1987). After first strand cDNA synthesis (e.g. with the Amersham first strand cDNA kit), DNA fragments encoding HC-V fragments and part of the long or short hinge region were amplified by PCR using specific primers: 
PstI
VH - 2B 5'-AGGTSMARCTGCAGSAGTCWGG-3' (SEQ. ID NO. 6)
S = C and G, M = A and C, R = A and G, W = A and T,

HindIII
Lam-07 5'-AACAGTTAAGCTTCCGCTTGCGGCCGCGGAGCTGGGGTCTTCGCTGTGGTGCG-3'
(short hinge) (SEQ. ID NO. 7)

HindIII
Lam-08 5'-AACAGTTAAGCTTCCGCTTGCGGCCGCTGGTTGTGGTTTTGGTGTCTTGGGTT-3'
(long hinge) (SEQ. ID NO. 8)

Upon digestion of the PCR fragments with *Pst*I (coinciding with codon 4 and 5 of the HG-V domain, encoding the amino acids L-Q) and *Bst*EII (located at the 3'-end of the HC-V gene fragments, coinciding with the amino acid sequence Q-V-T), the DNA fragments with a length between 300 and 400bp (encoding the HC-V domain, but lacking the first three and the last three codons) were purified via gel electrophoresis and isolation from the agarose gel.

### ii) Construction of Saccharomyces cerevisiae expression plasmids encoding llama HC-V domains

Plasmids pUR4547 and pUR4548 are *Saccharomyces cerevisiae* episomal expression plasmids, derived from pSY1 (Harmsen et al., Gene, 125: 115-123, (1993). From pSY1 the *Pst*I site, located in front of the GAL7 promoter was removed after partial digestion with *Pst*I, incubation with Klenow fragment and subsequent blunt end ligation. After transformation the desired plasmid could be selected on the basis of restriction pattern analysis. Subsequently, the *Bst*EII site in the Leu2 selection marker was removed by replacing the about 410bp *Afl*II/*Pfl*MI fragment with a corresponding fragment in which the *Bst*EII site was removed via a three step PCR mutagenesis, using the primers:

### PCR-A:

PflMI
BOLI 1    5'-GGGAATTCCAATAGGTGGTTAGCAATCG    (SEQ. ID NO. 9)

(BstEII)
BOLI 4    5'-GACCAACGTGGTCGCCTGGCAAAACG    (SEQ. ID NO. 10)

### PCR-B:

(BstEII)
BOLI 3   5'-CGTTTTGCCAGGCGACCACGTTGGTC   (SEQ. ID NO. 11)

AflII
BOLI 2   5'-CCCCAAGCTTACATGGTCTTAAGTTGGCGT   (SEQ. ID NO. 12)

PCR-A was performed with primers BOLI 1 and BOLI 4 and resulted in an about 130bp fragment with the *Pfl*MI restriction site at the 3'-end and the inactivated *Bst*EII site at the 5'-end. PCR-B was performed with primers BOLI 2 and BOLI 3 and resulted in an about 290bp fragment with the *Afl*II site at the 5'-end. The third PCR was with the fragments obtained from reaction A and B, together with the primers BOLI 1 and BOLI 2.

Finally, the about 1.8kb *Sac*I-*Hind*III fragment was replaced with synthetic fragments, having sequences as presented below, resulting the plasmids pUR4547 and pUR4548, respectively.

### - SacI/HindIII fragment of pUR4547

SacI                                                    (SEQ. ID NO. 13)
GAGCTCATCACACAAACAAACAAAACAAAATGATGCTTTTGCAAGCCTTCCCTT
1 ---------+---------+---------+---------+---------+---- 54
CTCGAGTAGTGTGTTTGTTTGTTTTGTTTTACTACGAAAACGTTCGGAAGGGAA
                              M  M  L  L  Q  A  F  L  F
                               |→      SUC2 ss   (SEQ. ID NO. 14)

                                                      PstI
TTCCTTTTGGCTGGTTTTGCAGCCAAAATATCTGCGCAGGTGCAGCTGCAGG
55------+---------+---------+---------+---------+----- 105
AAGGAAAACCGACCAAAACGTCGGTTTTATAGACGCGTCCACGTCGACGTCC
  L  L  A  G  F  A  A  K  I  S  A  Q  V  Q  L  Q  E
                                    |→

                    BstEII                        HindIII
AGTCATAATGAGGGACCCAGGTCACCGTCTCCTCATAATGACTTAAGCTT
106----+---------+---------+---------+---------+----- 155
TCAGTATTACTCCCTGGGTCCAGTGGCAGAGGAGTATTACTGAATTCGAA
  E  S  *  *  G  T  Q  V  T  V  S  S  *  *
   HC-V cassette                    ←|

and

### - SacI/HindIII fragment of pUR4548

SacI                                                    (SEQ. ID NO. 15)
GAGCTCATCACACAAACAAACAAAACAAAATGATGCTTTTGCAAGCCTTCCTTT
1 ---------+---------+---------+---------+---------+---- 54
CTCGAGTAGTGTGTTTGTTTGTTTTGTTTTACTACGAAAACGTTCGGAAGGAAA
                              M  M  L  L  Q  A  F  L  F
                              |→       SUC2 ss   (SEQ. ID NO. 16)

                                                    PstI
TCCTTTTGGCTGGTTTTGCAGCCAAAATATCTGCGCAGGTGCAGCTGCAGG
55-----+---------+---------+---------+---------+----- 105
AGGAAAACCGACCAAAACGTCGGTTTTATAGACGCGTCCACGTCGACGTCC
 L  L  A  G  F  A  A  K  I  S  A  Q  V  Q  L  Q  E
                                    |→

                        BstEII
AGTCATAATGAGGGACCCAGGTCACCGTCTCCTCAGAACAAAAACTCATC
106---+---------+---------+---------+---------+----- 155
TCAGTATTACTCCCTGGGTCCAGTGGCAGAGGAGTCTTGTTTTTGAGTAG
  S  *  *  G  T  Q  V  T  V  S  S  E  Q  K  L  I
  HC-V cassette                  ←|→        myc tail

HindIII
TCAGAAGAGGATCTGAATTAATGACTTAAGCTT
156----+---------+---------+-------- 188
AGTCTTCTCCTAGACTTAATTACTGAATTCGAA
S E E D L N * *
←|

Both plasmids contain the GAL7 promoter and PGK terminator sequences as well as the invertase (SUC2) signal sequence. In both plasmids the DNA sequence encoding the SUC2 signal sequence is followed by the first 5 codons, (encoding Q-V-Q-L-Q) of the HC-V domain (including the *Bst*II site), a stuffer sequence, the last six codons (encoding Q-V-T-V-S-S) of the HC-V domain. In pUR4547, this is followed by two stop codons, an *Afl*II and *Hind*III site. In pUR4548, this sequence is followed by eleven codons encoding the myc-tag, two stop codons, an *Afl*II and *Hind*III site.

Plasmids pUR4547 and pUR4548 were deposited under the Budapest Treaty at the Centraal Bureau voor Schimmelcultures, Baarn on 18th August 1997 with deposition numbers: CBS 100012 and CBS 100013, respectively. In accordance with Rule 28(4) EPC, or a similar arrangement from a state not being a contracting state of the EPC, it is hereby requested that a sample of such deposit, when requested, will be submitted to an expert only.

Upon digesting pUR4548 with *Pst*I and *Bst*EII, the about 6.4kb vector fragment was isolated and ligated with the *Pst*I-*Bst*EII fragments of about 350bp obtained as described above. After transformation of *S. cerevisiae*, via electroporation, transformants were selected from minimal medium agar plates (comprising 0.7% yeast nitrogen base, 2% glucose and 2% agar, supplemented with the essential amino acids and bases).

### iii) Screening for antigen specific HC-V domains

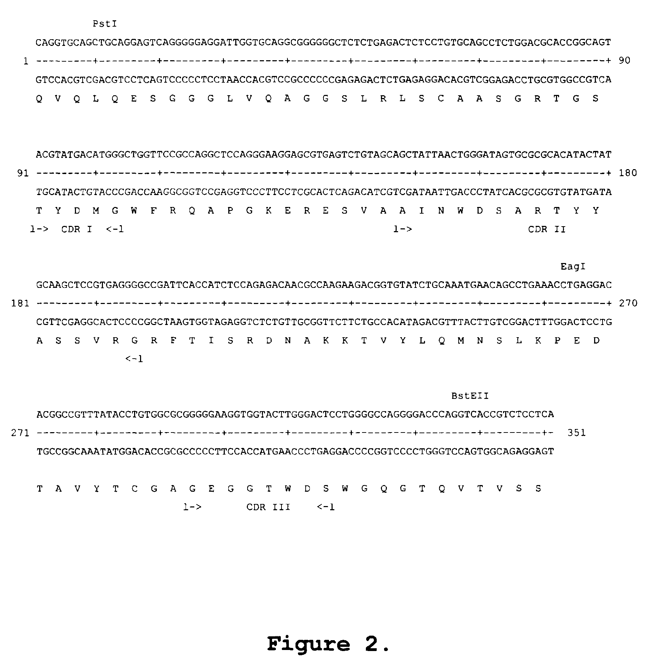

For the production of llama HC-V fragments with myc-tail, individual transformants were grown overnight in selective minimal medium (comprising 0.7% yeast nitrogen base, 2% glucose, supplemented with the essential amino acids and bases) and subsequently diluted ten times in YPGal medium (comprising 1% yeast extract, 2% bacto pepton and 5% galactose). After 24 and 48 hours of growth, the culture supernatant of the colonies was analysed by ELISA for the presence of HC-V fragments which specifically bind to the antigens hCG, RR6 in essential the same way as described above. In this case, however, the presence of specifically bound HC-V fragments was detected by incubation with monoclonal anti-myc antibodies, followed by incubation with poly-clonal rabbit-anti-mouse conjugate with alkaline phosphatase. In this way a number of anti-hCG and anti-RR6 HC-V fragments were isolated, which are:
anti-RR6:
   R9 pUR4640 (seeFigure 1) (SEQ. ID NO. 17, 18)
anti-hCG (alpha unit):
   H14 pUR4601 (see Figure 2) (SEQ. ID NO. 19, 20)

### c) Production of llama HC-V biheads by S. cerevisiae

### i) Construction of episomal expression plasmids encoding anti-hCG/anti-RR6 bispecific biheads

In the anti-hCG HC-V fragment H14 (anti-alpha-subunit), the *Pst*I site was removed and a *Xho*I site was introduced via PCR, using the primers:

MPG158WB

XhoI
5'-GAATTAAGCGGCCGCCCAGGTGAAACTGCTCGAGTCWGGGGGA-3'   (SEQ. ID NO. 21)

and

MPG159WB

BstEII
3'-CCCTGGGTCCAGTGGCAGAGGAGTGGCAGAGGAGTCTTGTTT-5'   (SEQ. ID NO. 22)

In this way the sequence:

PstI
CAG GTC CAG CTG CAG GAG TCT GGG (SEQ. ID NO. 23)
Q V Q L Q E S G

became

XhoI
CAG GTG AAA CTG CTC GAG TCW GGG (SEQ. ID NO. 24)
Q V K L L E S G

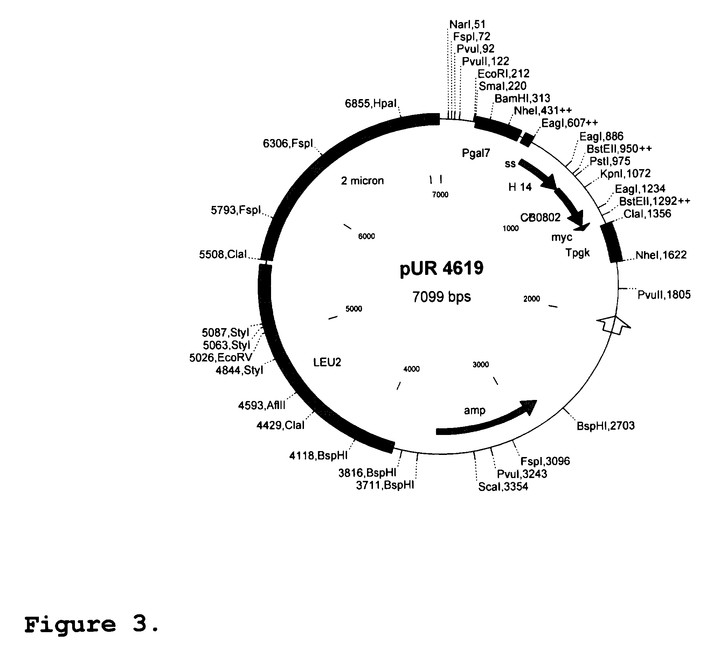

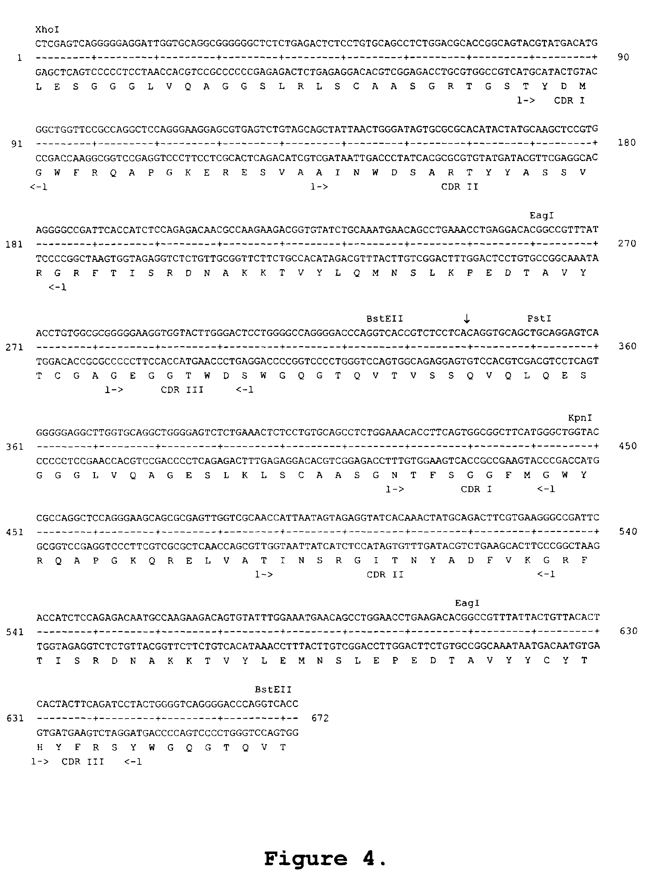

Upon digesting the PCR fragments with *Xho*I and *Bst*EII, the about 330bp fragments were purified via agarose gel electrophoresis and isolation from the gel. The fragments were cloned into pUR4421 (see Example 1 in WO 94/25591) which was digested with the same enzymes, resulting in pJS2 (H14). Subsequently, the about 420bp *Eag*I -*Hind*III fragment of pJS2 was isolated and ligated in the about 6.6kb EagI- *Hind*III vector fragment of the pSY1 plasmid of which the *Pst*I and *Bst*EII sites were removed as described above. The resulting plasmid pJS7, was digested with *Bst*EII and *Hind*III, after which the purified vector fragment was religated in the presence of a synthetic linker having the following sequence: 
BstEII                    PstI                    HindIII
 <-              MPG 160 WB (49)              ->  (SEQ ID NO. 25)
GGTCACCGTCTCCTCACAGGTGCAGCTGCAGGAGTCACTGTAATGACTTAAGCTT
---------+---------+---------+---------+---------+----- 55
CCAGTGGCAGAGGAGTGTCCACGTCGACGTCCTCAGTGACATTACTGAATTCGAA
      <-         MPG 161 WB (48)              ->
 V  T  V  S  S  Q  V  Q  L  Q  E  S  L  *  *  L  K  L
                                              (SEQ. ID NO. 26)
 resulting in plasmid pJS9. Finally, the plasmid was digested with *Pst*I and *Hind*III, after which the purified vector fragments of about 7.0kb were ligated with the *Pst*I -*Hind*III fragments of about 350bp of pUR4638 and pUR4640, encoding an anti-RR6 HC-V fragment denoted R9 followed by the myc-tail. The resulting *S. cerevisiae* episomal expression plasmid pUR4619 encodes a anti-hCG-anti-RR6 bispecific bihead preceded by the SUC2 signal sequence and followed by the myc-tail. pUR4619: SUC2 - H14 - R9 - myc (see Figures 3-4/SEQ. ID NO. 27, 28)

Upon digesting these plasmids with *Xho*I and partially with *Bst*EII, *Xho*I-*Bst*EII fragments of about 0.7kb can be isolated and subsequently cloned into the vector fragment of pUR4547 (digested with the same enzymes). In this way biheads can be obtained without the myc tail.

It will be appreciated that expression vectors can be constructed in which different promoter systems, e.g. the constitutive GAPDH promoter or different signal sequences, e.g. the mating factor prepro sequence are used.

### ii) Production of the HC-V biheads

After introducing the expression plasmid pUR4619 into *S. cerevisiae* via electroporation, transformants were selected from minimal medium agar plates as described in part b(ii) above. For the production of biheads, the transformants were grown overnight in selective minimal medium and subsequently diluted ten times in YPGal medium. After 24 and 48 hours of growth, samples were taken for Western blot analysis. For the immuno detection of the produced biheads via Western blot analysis, monoclonal anti-myc antibodies were used, followed by incubation with poly-clonal rabbit-anti-mouse conjugate with alkaline phosphatase.

### d) Anti-hCG/anti-RR6 bispecific biheads containing a linker peptide

### i) Construction of S. cerevisiae episomal expression plasmids encoding anti-hCG/anti-RR6 bispecific biheads containing a linker peptide

Between the H14 and the R9 encoding DNA fragments synthetic linkers were introduced encoding different linker peptides. To this end the about 50 bp long *Bst*EII-*Hind*III fragment of pJS7 (see Example 1 c(i) above) was replaced by an about 50 bp long *Bst*EII-*Hind*III fragment having the following sequence:

MVaJA

BstEII XbaI DraIII PstI HindIII
5′ GTCACCGTCTCTAGATGGCCACCAGGTGCAGCTGCAGGAGTCAACTTA 3′ (SEQ. ID NO. 29)

MVbJA

3′ GCAGAGATCTACCGGTGGTCCACGTCGAGCTCCTCAGTTGAATTCGA 5′
(SEQ. ID NO. 30)

This resulted in pSJ7a. In this plasmid the about 20 bp *Pst*I-*Hind*III fragment was replaced with the about 370 bp *Pst*I-*Hind*III fragment encoding the anti-RR6 HC-V fragment R9 and/with the myc-tail of pUR4G40 (see Example 1 c(i)) and resulting in pSJ7b.

Upon digesting plasmid pSJ7b with *Xba*I and *Dra*III the about 7 kb vector fragment was ligated with five synthetic oligo nucleotide linker fragments presented below:

MV01JA 5′ CTAGTGGTACTTCCGGTTCCCAG 3′ (SEQ. ID NO. 31)

MV02JA 3′ ACCATGAAGGCCAAGG 5′ (SEQ. ID NO. 32)

S G T S G S Q

MV03JA 5′ CTAGTTCTTCATCTGCTTCTGCCTCTTCAGCCCAG 3′ (SEQ. ID NO. 33)

MV04JA 3′ AAGAAGTAGACGAAGACGGAGAAGTCGG 5′ (SEQ. ID NO. 34)

S S S S A S A S S A Q

MV05JA 5′CTAGTGGTTCTCCAGGTTCACCAGGTCAG 3′ (SEQ. ID NO. 35)

MV06JA 3′ ACCAAGAGGTCCAAGTGGTCCA 5′ (SEQ. ID NO. 36)

S G S P G S P G Q

MV07JA 5′ CTAGTGCTACTACAACTGGTTCTTCACCAGGTCCAACTCAG 3′
(SEQ. ID NO. 37)

MV08JA 3′ ACGATGATGTTGACCAAGAAGTGGTCCAGGTTGA 5′
(SEQ. ID NO. 38)
S A T T T G S S P G P T Q

MV09JA 5′ CTAGTGCTAATCATTCTGGTAATGCTTCTCAG 3′ (SEQ. ID NO. 39)

MV10JA 3′ ACGATTAGTAAGACCATTACGAAGA 5′ (SEQ. ID NO. 40)

S A N H S G N A S Q

The oligonucleotide linker fragments encode the last amino acid of the N-terminal HC-V fragment (S) and the first amino acid of the C-terminal HC-V fragment, intersected by the connecting linker peptide. This resulted in plasmids pUR5330 to 5334, respectively.

After transformation of *S. cerevisiae* with these plasmids, the production levels of the biheads were determined via Western blot analysis and a anti-hCG ELISA using anti-myc mAb for detection of the bound bihead (see Example 1 b(iii). Production levels are presented in Table 2 below:

**Table 2**

| Plasmid | Linker | Production level (mg/l) |
|---|---|---|
| pUR4619 | None | 11 |
| pUR5330 | S-**G-T-S-G-S**-Q | 36 |
| pUR5331 | S-**S-S-S-A-S-A-S-S-A**-Q | 49 |
| pUR5332 | S-**G-S-P-G-S-P-G**-Q | 33 |
| pURS333 | S**-A-T-T-T-G-S-S-P-G-P-T**-Q | 56 |
| pUR5334 | S-**A-N-H-S-G-N-A-S**-Q | 51 |

The production levels of the biheads in which the two HC-V domains are separated by a linker peptide (consisting of between 5 and 11 amino acids) were found to be 3 to 5 times higher as found for the bihead in which the two HC-V fragments are connected without a peptide linker.

Finally, the bispecificity of the biheads was demonstrated as follows:

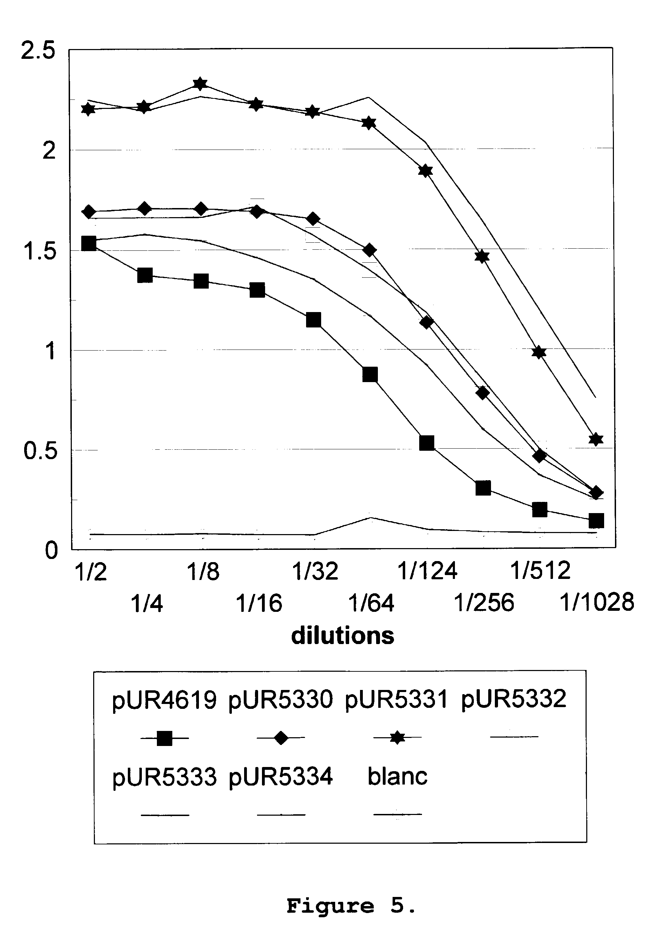

PINs coated with hCG were incubated with (diluted) medium samples. Subsequently, the PINs were incubated with a RR6-alkaline phosphatase conjugate, in which the azo-dye RR6 was coupled to the alkaline phosphatase via its reactive triazine group. Finally, the alkaline phosphatase enzyme activity was determined after incubation of the PINs with p-nitro-phenyl phosphate and the optical density was measured at 405nm (see Figure 5).

### 1.2 Purification of Llama Bi-heads with Various Linkers from S. cerevisiae Culture Media

A 5 ml column of recombinant Protein A Fast Flow Sepharose (Amersham Pharmacia Biotech) was equilibrated by washing with 10 column volumes of wash buffer (10 mM potassium phosphate, pH 6), at a flow rate of 2 ml/min. The bi-head fermentation broth was loaded at 2 ml/min in an upwards direction. After loading, the column was washed with wash buffer until the OD₂₈₀ reached the baseline. Elution was carried out with a linear gradient of 0 - 40 mM citric acid pH 2.5 in the reverse direction, collecting 4 ml fractions into tubes containing 400 µl of a neutralising agent (1M Tris.Cl, pH 8.5) in order to minimise the effects of the acid. Peak fractions were checked for purity by running on a 12% SDS-PAGE Ready Gel (Bio-Rad) under standard denaturing conditions. Staining was with GelCode Blue (Pierce & Warriner). The fractions were concentrated using Macrosep centrifugal concentrators (3 kDa molecular weight cut-off, Pall Filtron Corp.) then buffer exchanged into 10mM potassium phosphate, pH 6 using PD-10 columns (Amersham Pharmacia Biotech). The final purity of the sample was determined by carrying out a UV scan from 400 - 220 nm and using the value at 280 nm to determine an accurate concentration. The samples were then aliquoted into vials, frozen, freeze dried and stored until required.

### 1.3 Preparation of a Reactive Red 6-Bovine Serum Albumin Conjugate

A solution of Reactive Red 6 (RR6) was made up at 10 mg/ml in phosphate buffered saline (PBS). A solution of bovine serum albumin (BSA) was made up at 10 mg/ml in PBS. 200 µl of the RR6 solution was added to 800 µl of the BSA solution and the resulting solution was mixed in an end over end rotary mixer for 2 hours at room temperature. RR6 that had conugated to BSA was separated from free RR6 by addition of the reaction mixture (1 ml) to a PD10 column (Pharmacia) previously washed with 10 ml of PBS containing 0.1% sodium azide (PBSA). The column was then eluted by addition of PBSA (5 ml) and 1 ml aliquots were collected. The RR6-BSA conjugate eluted in fractions 4 and 5. These were pooled and the concentration of protein was determined using a BCA protein test and the concentration adjusted to 2 mg/ml with PBSA.

### 1.4 Adsorption of Latex with Reactive Red 6-Bovine Serum Albumin Conjugate

Duke blue latex was adsorbed with the RR6-BSA conjugate as follows:

To 950 µl of 10 mM borate buffer, 0.01 % merthiolate, pH 8.5 (buffer B) a 50 µl aliqot of Duke blue latex (10 % solids) was added and mixed by inverting. The diluted latex was centrifuged at 8000 g for 10 minutes at room temperature, the supernatant removed and the pellet vortexed briefly. The pellet was resuspended in 900 µl of buffer B and to this 100 µl of the previously prepared RR6-BSA conjugate was added. The latex solution was sonicated for 10 s using a sonic probe. The solution containing the latex was mixed for 2 h at room temperature and then centrifuged (8000 g, 10 min at room temperature). The latex pellet was washed by resuspending in 1 ml of buffer B and centrifuged once more (8000 g, 10 min at room temperature). The pellet was then resuspended in 1 ml buffer B ready for use.

### 1.5 Analysis of Llama Bi-head Self Assembling on Reactive Red 6-Bovine Serum Albumin Adsorbed Latex

The llama bi-heads were tested by self assembling onto RR6-BSA adsorbed latex and detection of hCG in a rapid assay technology (RAT) format. This was performed by mixing the llama bi-head (5 µl of a 0.1 mg/ml solution) with RR6-BSA adsorbed latex (5 µl of 0.1 % solids) in 10 µl of PBSA to which hCG (5 µl of various concentrations) was added.

The resulting solution was added to the bottom of a nitrocellulose strip (6 mm wide x 30 mm long) on which a monoclonal antibody recognising hCG had been adsorbed by plotting in a line (2.5 mg/ml) mid way up the strip. The latex-bi-head-hCG solution was allowed to flow up the nitrocellulose strip by capillary action and the strip was then washed by applying PBSA (25 µl) to the bottom of the strip. The amount of latex, captured at the plotted antibody line on the nitrocellulose strip, was quantified by measuring the absorbance through the strip.

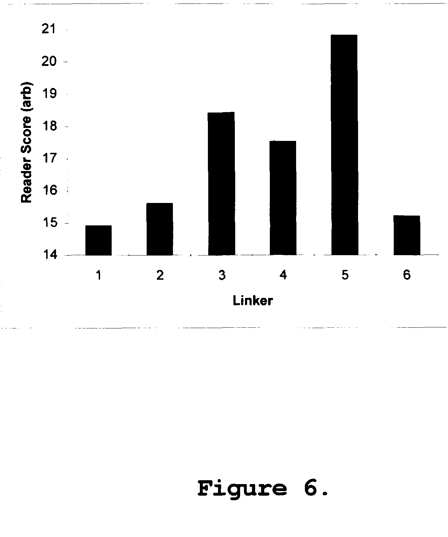

Figure 6 shows that the llama bi-heads with linkers 3, 4 and 5 gave the highest response in RAT assays. These linkers are structurally more ordered than the comparative examples, flexible linkers 2 and 6 and result in more hCG and more latex captured in the assay. The more ordered linkers promote the correct orientation of the binding domains to achieve more optimal binding than when no linker is used. Linker 3 is derived from CWP1 and Linker 5 from CBH1P.

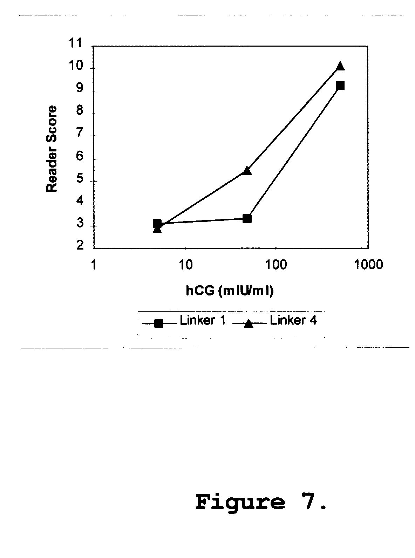

Synthetic linkers with some order (linker 4 containing 2 proline residues) can offer increased sensitivity in assays than those with little order (linker 2). Figure 7 shows that the bi-head with linker 4 can detect lower amounts (50 mIU/ml) of hCG than the bi-head with linker 2 and, hence, give a more sensitive assay for hCG.

## Claims

1. Use of a polypeptide group, the amino acid sequence of which group confers restricted conformational flexibility, as a linking group to link binding units in a multivalent binding protein.

2. Use according to claim 1 wherein the polypeptide linking group comprises from 4 to 30 amino acid residues.

3. Use according to claim 1 or 2 wherein the linking group comprises one or more proline residues.

4. Use according to claim 1 or 2 wherein the linking group comprises an amino acid sequence selected from:
S-S-S-A-S-A-S-S-A,
G-S-P-G-S-P-G, or
A-T-T-T-G-S-S-P-G-P-T.

5. A multivalent binding protein comprising a plurality of binding units linked by means of intervening polypeptide linker groups, the amino acid sequence of which linker group confers restricted conformational flexibility.

6. A protein according to claim 5 wherein the binding units comprise heavy chain variable domains derived from an immunoglobulin naturally devoid of light chains.

7. A protein according to claim 5 or claim 6 wherein the antigen binding units comprise heavy chain variable domains derived from a Camelid immunoglobulin.

8. A protein according to any one of claims 5 to 7 comprising a bivalent antigen binding protein.

9. A protein according to any one of claims 5 to 8 wherein the linker group comprises from 4 to 30 amino acid residues.

10. A protein according to any one of claims 5 to 9 wherein the linker group comprises one or more proline residues.

11. A protein according to any one of claims 5 to 9 wherein the linker group comprises an amino acid sequence selected from:
S-S-S-A-S-A-S-S-A,
G-S-P-G-S-P-G, or
A-T-T-T-G-S-S-P-G-P-T.

12. Nucleotide sequences encoding for a multivalent binding protein of any one of claims 5 to 11.

13. An expression vector comprising a nucleotide sequence according to claim 12.

14. A host cell transformed with a vector according to claim 13.
